# EUROPEAN PATENT SPECIFICATION

(11) **EP 1 829 872 B1**
(45) Date of publication and mention of the grant of the patent: **29.07.2015**
(21) Application number: 05809586.0
(22) Date of filing: 25.11.2005
(51) Int. Cl.: C07D 403/12, C07D 209/08, C07B 61/00

(54) **PROCESSES FOR PRODUCTION OF INDOLE COMPOUNDS**
VERFAHREN ZUR HERSTELLUNG VON INDOLVERBINDUNGEN
PROCEDES DE PRODUCTION DE COMPOSES D'INDOLE

(30) Priority: 25.11.2004 JP 2004340253
(43) Date of publication of application: 05.09.2007
(73) Proprietor: Nissan Chemical Industries, Ltd., Chiyoda-ku, Tokyo 101-0054 (JP)
(72) Inventor: FUKUDA, Kenzo, Yamaguchi 754-1212 (JP); KONDO, Yasuo c/o Nissan Chemical Ind., Ltd.,, SanyoOnoda-shi, Yamaguchi 7560093 (JP); MAKABE, Takahiro c/o Nissan Chemical Ind., Ltd.,, SanyoOnoda-shi, Yamaguchi 7560093 (JP); TANAKA, Norio c/o Nissan Chemical Ind., Ltd.,, Funabashi-shi, Chiba 2748507 (JP); UTSUNOMIYA, Tomohisa c/o Nissan Chem. Ind., Ltd.,, Funabashi-shi, Chiba 274850 (JP); SAKURAI, Yasuhiro c/o Nissan Chem. Ind. Ltd.,, Funabashi-shi, Chiba 2748507 (JP)
(74) Representative: Hoffmann Eitle
(86) International application number: PCT/JP2005/021720
(87) International publication number: WO 2006/057354

(56) References cited:
- JP-A- 2004 083 559
- RAUCHER S. ET AL: 'Synthesis of Substitution Indoles via Meerwein Arylation.' JOURNAL OF ORGANIC CHEMISTRY. vol. 48, 1983, pages 2066 - 2069, XP002970217
- MEJETICH G. ET AL: 'Electrophilic Aromatic Bromination Using Bromodimethylsulfonium Bromide Generated in Situ.' JOURNAL OF ORGANIC CHEMISTRY. vol. 62, 1997, pages 4321 - 4326, XP002103720
- STRIVASTAVA S.K. ET AL: 'Novel site-specific one-step bromination of substituted benzenes.' CHEMICAL COMMUNICATIONS. vol. 23, 1996, pages 2679 - 2680, XP009031635

## Description

### Technical Field

The present invention relates to a process for producing indole compound useful as several fine chemical intermediates represented by physiologically active substances such as pharmaceuticals and agrochemicals, etc.

### Background Art

As a process for producing indole compound, the followings are known. There is an example in N-o-tolyl-acetamide is reacted with barium oxide at 360°C to obtain 2-methyl indole (Patent Document 1). Similarly, there are also examples in which sodium amide (Non-patent Document 1) or sodium methoxide (Non-patent Document 2) is used, but these examples require a high temperature and produce a large amount of by-product and the yield is not so high.

Although there is an example in which a phenyl hydrazone of acetone is reacted with sodium hydroxide at 240°C to obtain 2-methyl indole, it produces a large amount of by-product and has a low yield (Non-patent Document 3).

Further, although it is known to produce 2-methyl indole by reacting 2-nitro-1-(2-nitrophenyl) propene with hydrogen in the presence of 10% palladium catalyst supported on active carbon, the yield is 81 % (Non-patent document 4).

In addition, although it is reported to obtain 2-methyl indole in a yield of 64% by reacting aniline with tris(2-hydroxypropyl) amine hydrochloride in the presence of tin dichloride, ruthenium trichloride and triphenyl phosphine at 180°C, the yield is low (Non-patent Document 5).

The process for producing indole compound from 2-nitrobenzylcarbonyl compound includes for example, a report in which 2-nitrophenyl acetone is reduced with iron in the presence of acetic acid and sodium acetate to obtain 2-methyl indole in a yield of 68% (Non-patent Document 6). However, the yield in the process is low. In addition, there is a report in which 4-fluoro-2-nitrophenyl acetone is reacted with zinc in an acetic acid aqueous solution to obtain 6-fluoro-2-methyl indole in a yield of 95% (Patent Document 2), and the like. However, this process discharges a large amount of zinc oxide as waste in post-treatment, and influence adverse effect on environment. In addition, although there is a report stating that a catalytic reduction in the presence of a catalyst such as palladium, Raney nickel, platinum or the like also provides similar products (Patent Document 2), it does not disclose any working examples corresponding thereto.

In the process in which a reducing catalyst such as a noble metal supported catalyst and a hydrogen donor are used, examples that 2-substituted indole compound is produced in one step and a high yield are rare. Actually, when 4-fluoro-2-nitrophenylacetone is reduced with hydrogen gas in the presence of a palladium catalyst supported on active carbon, the yield of 6-fluoro-2-methyl indole is about 70% as 6-fluoro-2-methyl indoline is formed as a by-product. This is because 1-hydroxy-2-methyl indole produced as a reaction intermediate has a relation of tautomerism with 2-methyl indolenine N-oxide, and this 2-methyl indolenine N-oxide is further reduced to form 6-fluoro-2-methyl indoline

The synthetic example of 1-hydroxy-2-alkyl indole as a reduction intermediate includes an example in which 2-nitrophenyl acetone is reduced with zinc and ammonium chloride to synthesize 1-hydroxy-2-methyl indole (Non-patent Document 7), and a synthetic example by starting from electrochemical α-(o-hydroxyaminophenyl) propene (Non-patent Document 8). In addition, it is known that 1-hydroxy-2-methyl indole has a relation of tautomerism with 2-methyl indolenine N-oxide (Non-patent Documents 9 and 10).

Next, the examples of the acylation include an example in which 1-acetoxy-2-phenyl indoles or 1-benzoyloxy-2-phenyl indoles are synthesized from 1-hydroxy-2-phenyl indoles by use of acetic anhydride and benzoyl chloride (Non-patent Document 11), and a synthetic example of 1-acetoxy-2-methyl indole (Non-patent Document 12).

Further, the examples of reductive reaction from the acylated form include an example in which 2-phenyl indole or 3-cyano-2-phenyl indole is formed by reducing 1-benzoyloxy-2-phenyl indole or 1-acetoxy-3-cyano-2-phenyl indole with an active carbon supported palladium catalyst in ethanol (Non-patent Document 13). However, this process requires many steps for producing the aimed indole compounds from 2-nitrobenzyl carbonyl compound as a starting material.

There is only one example in which reduction in one step and a high yield is carried out in by using an active carbon-supported 5% palladium and hydrogen in the presence of sodium acetate and acetic anhydride (Patent Document 3). However, the catalyst used in this method is expensive and dangerous, and therefore tedious procedures for recovery and re-use lead to an increase in production cost. As mentioned above, thus far there is no production method that any indoles can be produced by using inexpensive agents and simple procedures.

The halogenation at 3-position of indole ring is generally carried out with a halogen or hypohalogenous acid, etc. In case where the halogen is used, as a solvent such as toluene or chlorobenzene, etc. causes halogenation, it is required to use a solvent that does not receive any chlorination. In addition, in case where the halogen is used, it is apt to form dihalogeno indole (Non-patent Documents 14 and 15).

On the other hand, although hypohalogenous acid can prevent the halogenation of solvent, the formation of dihalogeno indole causes a lowering in yield (Non-patent Document 16). Therefore, it requires a step for forming the aimed 3-halogeno indole by reducing dihalogeno indole with a reducing agent such as sodium hydrogen sulfite, sodium sulfite or the like, and it becomes a tedious production method in an industrial aspect (Patent Document 3). Further, there are examples in which halogenated trimethyl silane, halogenated succinimide or halogenated copper is used (Patent Documents 4 and 5, Non-patent Documents 17, 18 and 19). However, these methods have a problem that expensive agents are used and a large amount of waste is generated.

Therefore, in the industrial production of 3-halogeno indole, the method of halogenation that does not cause these problems is required. The example of such a halogenation includes an example in which the reaction is carried out by using benzene or the like under a mild condition by use of a combination of dialkyl sulfoxide such as dimethyl sulfoxide, and a hydrogen halide acid such as hydrochloric acid or hydrobromic acid (Non-patent Documents 20 and 21). However, there is no example in which indole compounds are produced.
Thus, it is desired to provide an inexpensive halogenation method of indole compounds in a simple procedure that does not halogenize the solvent nor cause dihalogenation.

Although the method in which an indole compound and 1-(N,N-dimethylsulfamoyl)-3-chlorosulfonyl-1,2,4-triazole are reacted to produce a sulfamoyl triazole compound is known (Patent Document 4), it requires to use expensive potassium tertiary butoxide and sodium hydride (Patent Documents 4 and 5). In addition, there is an example in which a sodium salt of an indole compound is once produced, and then it is reacted with 1-(N,N-dimethyl sulfamoyl)-3-chlorosulfonyl-1,2,4-triazole (Patent Document 3). However, it requires procedures that the reaction solvent from the preceding step is distilled off and then reacted in an ether solvent such as diglyme or the like, and further after the reaction the ether solvent is distilled off and extracted with other solvent again. Therefore, it cannot be said that this is an inexpensive industrial production method.

On the other hand, as reactions of an indole compound with a benzene sulfonyl chloride compound, many examples in which the reaction is carried out in one step by use of inexpensive sodium hydroxide are known (Non-patent Documents 15 and 22). The methods that are carried out in simpler procedures and a higher yield as mentioned above are also required for the reaction of an indole compound with 1-(N,N-dimethylsulfamoyl)-3-chlorosulfonyl-1,2,4-triazole.
Some production methods other than the above-mentioned methods are reported (Patent Documents 6, 7 and 8).
Patent Document 1: DE Patent No. 262327 Specification
Patent Document 2: JP-A-47-38963 (1972)
Patent Document 3: JP-A-2004-083559 (2004)
Patent Document 4: JP-A-2000-302781 (2000)
Patent Document 5: JP-A-2001-187786 (2001)
Patent Document 6: JP-A-2001-247567 (2001)
Patent Document 7: JP-A-2002-241364 (2002)
Patent Document 8: International Patent Publication WO99/21851 pamphlet
Non-patent Document 1: Bull. Soc. Chim. Fr., 4, p. 1039 (1924)
Non-patent Document 2: Org. Syn., 27, p. 94 (1942)
Non-patent Document 3: Chem. Ber. 81, 266, p. 270 (1948)
Non-patent Document 4: Heterocycles, 55, p. 95 (2001)
Non-patent Document 5: Tetrahedron, p. 3321 (2001)
Non-patent Document 6: J. Org. Chem., 48, p. 2066 (1983)
Non-patent Document 7: Bull. Soc. Chim. Fr., p. 1296 (1967)
Non-patent Document 8: Bull. Soc. Chim. Fr., p. 121 (1974)
Non-patent Document 9: J. Chem. Soc., p. 1067 (1970)
Non-patent Document 10: Spectrochim. Acta, 23, p. 717 (1967)
Non-patent Document 11: J. Chem. Soc., p. 3466 (1960)
Non-patent Document 12: Bull. Soc. Chim. Fr., p. 3040 (1973)
Non-patent Document 13: J. Chem. Soc., p. 3466 (1960)
Non-patent Document 14: Synlett, p. 705 (2003)
Non-patent Document 15: Syn. Com., 34, p. 1325 (2004)
Non-patent Document 16: J. Org. Chem., 46, p. 2054 (1981)
Non-patent Document 17: J. Chem. Research, Synopses, 6, p. 182 (1989)
Non-patent Document 18: Tetrahedron Letters, 27, p. 1051 (1986)
Non-patent Document 19: J. Chem. Soc. Perkin Trans. 1, p. 2305 (1986)
Non-patent Document 20: J. Org. Chem., 62, p. 4321 (1997)
Non-patent Document 21: Chem. Com., p. 2679 (1996)
Non-patent Document 22: Tetrahedron Letters, 28, p. 3432 (1987)

### Disclosure of the Invention

### Problem to be solved by the Invention

The problem to be solved by the invention is to provide a process for producing indole compound that is industrially advantageous and inexpensive. Means for solving the Problem

The present inventors eagerly investigated in order to solve the above-mentioned problem. As a result of it, they found that indole compounds can be produced in one step and a good yield by using an acylating agent and a base together in the reduction of 2-nitrobenzylcarbonyl compound with a metal and an acid, and they also found an industrially advantageous and novel process for producing a halogeno compound and a sulfamoyl triazole compound of the indole compound, and they completed the present invention.

That is, the present invention relates to the following [1] to [10]
[1] A process for producing an indole compound of formula (2-2) 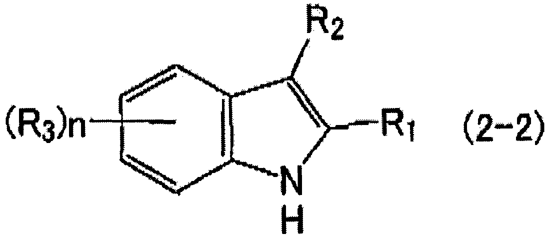 wherein R₁ is a hydrogen atom, a halogen atom, an optionally substituted alkyl group, or a phenyl group. R₂ is a bromine atom, R₃ is an optionally substituted alkyl group, a phenyl group, an alkoxy group, an alkoxycarbonyl group, or a halogen atom, and n is an integer of 0 to 4,
   comprising reacting an indole compound of formula (2-1) 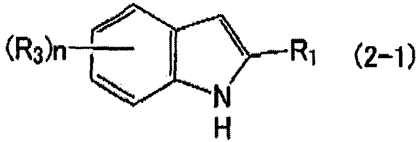 wherein R₁, R₃ and n have the same meaning as above,
   with a hydrogen halide acid and a sulfoxide compound, wherein
   the hydrogen halide acid is hydrobromic acid and
   the sulfoxide compound is dimethylsulfoxide.
[2] The process for producing an indole compound of formula (2-2) according to [1], further comprising reducing a 2-nitrobenzylcarbonyl compound of formula (1) 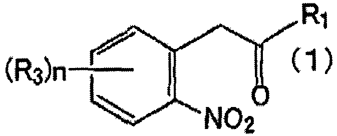 wherein R₁, R₃ and n have the same meaning as in claim 1,
   with a metal and an acid in the presence of an acylating agent and a base to produce the indole compound of formula (2-1).
[3] A process for producing a sulfamoyl triazole compound of formula (3) 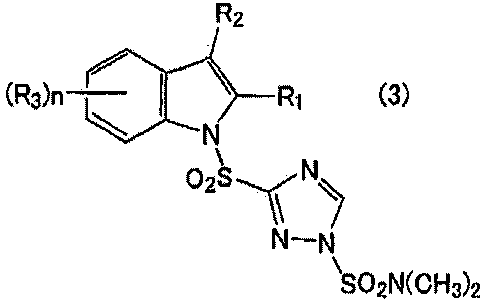 wherein R₁ is a hydrogen atom, a halogen atom, an optionally substituted alkyl group, or a phenyl group. R₂ is a bromine atom, R₃ is an optionally substituted alkyl group, a phenyl group, an alkoxy group, an alkoxycarbonyl group, or a halogen atom, and n is an integer of 0 to 4,
   comprising producing the indole compound of formula (2-2) by the process according to [1] or [2] and reacting the indole compound of formula (2-2) directly with 3-chlorosulfonyl-1-(N,N-dimethylsulfamoyl)-1,2,4-triazole in the presence of a phase transfer catalyst and a base.
[4] The process for producing an indole compound (2-2) according to [2], wherein the acylating agent is an organic acid anhydride
[5] The process for producing an indole compound (2-2) according to [2], wherein the acylating agent is acetic anhydride.
[6] The process for producing an indole compound (2-2) according to [2], wherein the base is an alkali metal salt or a hydroxide of alkali metal.
[7] The process for producing an indole compound (2-2) according to [2], wherein the base is an organic acid salt, carbonate, hydrogencarbonate or hydroxide of alkali metal.
[8] The process for producing an indole compound (2-2) according to [2], wherein the metal is iron.
[9] The process for producing an indole compound according to [1] or [2], wherein the indole compound of formula (2-2) is 3-bromo-6-fluoro-2-methylindole.
[10] A process for producing 3-bromo-6-fluoro-2-methyl indole, characterized by reacting 2-methyl-6-fluoroindole with hydrobromic acid and a sulfoxide compound.

The process for producing the indole compound of formula (1) from 2-nitrobenzylcarbonyl compound of formula (3) will be described in further detail. The present invention relates to a process for producing an indole compound of formula (2) 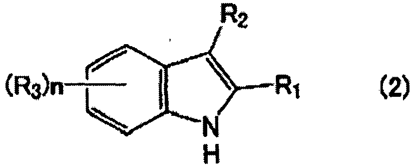 wherein R₁ is a hydrogen atom, an optionally substituted alkyl group, or a phenyl group, R₂ is a bromine atom, R₃ is an optionally substituted alkyl group, a phenyl group, an alkoxy group, an alkoxycarbonyl group, or a halogen atom, and n is an integer of 0 to 4, characterized in that the reduction of 2-nitrobenzylcarbonyl compound of formula (1) 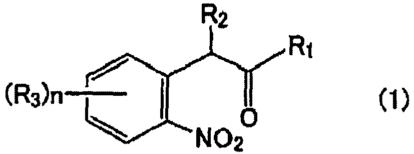 with a metal and an acid is carried out in the presence of an acylating agent and a base, and thereby 1-hydroxyindole of formula (4) 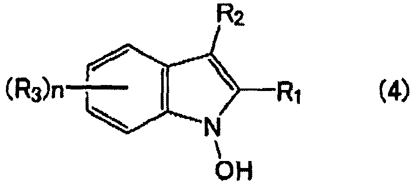 that is an intermediate, and indolenine N-oxide of formula (5) 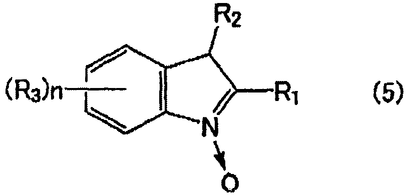 that is a tautomer thereof are acylated to form 1-acyloxy indole of formula (6) 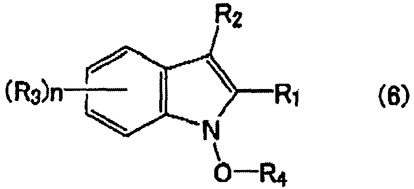 wherein R₄ is an acyl group of the acylating agent, and the indole compound of formula (2) is produced through the 1-acyloxy indole of formula (6). The process produces little indoline compounds being reduction by-products, and can produce indole compounds in a high yield.

The halogenation characterized by reacting the indole compound of formula (2) (in which R₂ is hydrogen atom) with hydrobromic acid and dimethyl sulfoxide compound can avoid problems that by-products such as dihalides are formed, and can be carried out in one step and a good yield, and thus the production process is improved in operation properties and very advantageous in industrial aspect.

In addition, the process for producing the compound of formula (3) by reacting the indole compound of formula (2) (in which R₂ is a bromine atom) directly with 3-chlorosulfonyl-1-(N,N-dimethylsulfamoyl)-1,2,4-triazole in the presence of a phase transfer catalyst and a base can be carried out also in a solvent other than ether solvents. Therefore, the process obviates a step for displacing solvent, and is very advantageous in industrial aspect.

### Effect of the Invention

The production process of the present invention is excellent in operating properties. Therefore, the production process can produce indole compounds industrially and in a good yield.

### Best Mode for Carrying Out the Invention

As the compounds to which the present invention is applied, 2-nitrobenzylcarbonyl compounds of formula (1) and indole compounds of formula (2) include compounds wherein R₁ is a hydrogen atom, a halogen atom, an optionally substituted alkyl group or a phenyl group, R₂ is a bromine atom, R₃ is an optionally substituted alkyl group, a phenyl group, an alkoxy group, an alkoxycarbonyl group or a halogen atom, and n is an integer of 0 to 4, or compounds wherein R₁ is a hydrogen atom or an optionally substituted alkyl group, R₂ is a bromine atom, R₃ is a halogen atom, and n is an integer of 0 or 1, or compounds wherein R₁ is methyl group, R₂ is a bromine atom, R₃ is fluorine atom, and n is an integer of 0 or 1.

The 2-nitrobenzylcarbonyl compounds of formula (3) being a starting material of the present invention is produced by any known methods. For example, the compounds include 2-nitrophenylacetone (Tetrahedron Lett., 42, p. 1387 (2001)), 1-(4-chloro-2-nitrophenyl)acetone (Chem. Pharm. Bull., 17, p. 605 (1969), and 1-(4-fluoro-2-nitrophenyl)acetone (JP-A-47-38947 (1972)).

Agents and reaction condition used in reducing the 2-nitrobenzylcarbonyl compounds are as follows. As the acylating agent, organic acid anhydrides having acyl group that is reaction active are effective, and preferably acetic anhydride, trifluoroacetic anhydride, propionic anhydride, butyric anhydride, capronic anhydride, crotonic anhydride, maleic anhydride, benzoic anhydride, succinic anhydride or the like, or mixed acid anhydrides synthesized from acetic anhydride and formic acid, and the like. A mixture of the above-mentioned compounds can be used. Among them, acetic anhydride is preferable from the viewpoint of economic efficiency.
The used amount of the acylating agent is generally 0.01 to 10 mol, preferably 0.5 to 5 mol based on 2-nitrobenzylcarbonyl compound.

The base includes organic bases such as amine or pyridine, etc., or alkali metal salts such as organic acid salt, carbonates, hydrogencarbonates, phosphates and sulfites or the like of alkali metal, alkaline earth metal salts such as organic acid salt, carbonates, hydrogencarbonates, phosphates and sulfites or the like of alkaline earth metal, inorganic bases such as hydroxides or oxides of alkali metal, and hydroxides or oxides of alkaline earth metal, etc., preferably includes alkali metal salts and hydroxides of alkali metal, or preferably includes organic acid salts of alkali metal or bases such as carbonates, hydrogencarbonates or hydroxides of alkali metal, etc. that form organic acid salts of alkali metal by reacting with organic acid anhydrides or organic acids in the reaction solution. Among them, sodium formate, potassium formate, sodium acetate, potassium acetate, sodium propionate, potassium propionate, sodium carbonate, sodium hydrogencarbonate, potassium carbonate, potassium hydrogencarbonate, sodium hydroxide, potassium hydroxide, calcium phosphate and the like are particularly preferable. These bases can be also used in a mixture.
The used amount of the base is preferably 0.01 to 5 mol, more preferably 0.1 to 2 mol based on 2-nitrobenzylcarbonyl compound.

As the metal, metals such as iron, zinc, tin, magnesium or the like that is generally used for reduction are preferable, further iron that is inexpensive and has a little influence on the environment is preferable from the viewpoint of economic efficiency, In addition, metal compounds such as iron sulfate or tin (II) chloride can be used.
The used amount of the metal is preferably 1 to 10 mol, further preferably 1.5 to 5 mol based on 2-nitrobenzylcarbonyl compound.
The shape of the metal or the particle size thereof may affect the reaction rate. In case where iron is used, there are iron powders such as reduced iron, electrolytic iron and the like, other than general iron powders, and any iron powders can be used irrespective of the particle size in the present reaction.

The acid includes organic acids such as formic acid, acetic acid, propionic acid, oxalic acid, succinic acid or the like, inorganic acids such as hydrochloric acid, sulfuric acid or the like. Among them, acetic acid containing no water is preferable also from the viewpoint of economic efficiency. When the acid contains water, it may cause hydrolysis of the acylating agent and thus provide no desirable effect.
The used amount of the acid is 1 to 20 mol, and preferably 3 to 10 mol based on 2-nitrobenzylcarbonyl compound.

The solvent is not specifically limited so long as it is an inert solvent for the present reaction, and for example includes aromatic solvents such as benzene, toluene, xylene and the like, ethers such as diethyl ether, dibutyl ether, tetrahydrofuran, 1,4-dioxane, dimethoxy ethane, diethylene glycol dimethyl ether and the like, esters such as methyl formate, ethyl formate, methyl acetate, ethyl acetate, butyl acetate, ethyl propionate and the like, ketones such as acetone, methyl ethyl ketone, methyl isobutyl ketone, cyclohexanone and the like, hydrocarbons such as hexane, heptane, octane, nonane and the like, polar solvents such as N,N-dimethylformamide, N,N-dimethylacetamide, N-methylpyrrolidone and the like. These solvents can be also used in a mixed solvent.
The used amount of the solvent is preferably 1 to 20 times, and further preferably 3 to 10 times that of 2-nitrobenzylcarbonyl compound.

The reaction for producing an indole compound from 2-nitrobenzylcarbonyl compound is carried out by reacting a mixture of 2-nitrobenzylcarbonyl compound, an acylating agent, a base, a metal, an acid and a solvent. Industrially preferable processes include a process in which an acid is added in a mixture of 2-nitrobenzylcarbonyl compound, an acylating agent, a base, a metal and a solvent at a reaction temperature at a rate that does not affect the reaction.
The reaction temperature generally ranges from a low temperature less than room temperature to some hundreds of temperature, and preferably ranges from room temperature to the boiling point of the reaction solvent.
The reaction can be carried out under a pressure from normal pressure to a high pressure such as 100 kg/cm² or the like, or under reduced pressure, and preferably under normal pressure.

The treatment of the reaction solution after reaction is carried out by removing metal oxides or unreacted metals by filtration, and then washing the reaction solution with water. The treatment can provide a solution containing indole compounds. When it is difficult to filtrate the solution, the filtration becomes easy by adding hydrochloric acid or sulfuric acid, etc. and thus dissolving the metal oxides.
In addition, the agents used in the reaction and by-products or the like can be removed by washing with alkaline aqueous solution such as sodium hydroxide or the like, or acidic aqueous solution such as sulfuric acid, hydrochloric acid or the like, if necessary.

The agents and reaction conditions used in the reaction of the indole compound of formula (2) in which R₂ is hydrogen atom with hydrobromic acid and dimethyl sulfoxide are as follows.
The hydrobromic acid includes hydrogen bromide, and the aqueous solution thereof, and hydrobromic acid aqueous solution is preferable.
The used amount of the hydrogen halide acid is generally 0.1 to 5 mol, preferably 1 to 2 mol based on the indole compound.
The used amount of the sulfoxide compound is generally 0.01 to 5 mol, preferably 0.1 to 2 mol based on the indole compound.

The solvent is not specifically limited so long as it is an inert solvent for the present reaction, and for example includes aromatic solvents such as benzene, toluene, xylene, chlorobenzene and the like, ethers such as diethyl ether, dibutyl ether, tetrahydrofuran, 1,4-dioxane, dimethoxy ethane, diethylene glycol dimethyl ether and the like, esters such as methyl acetate, ethyl acetate, butyl acetate, ethyl propionate and the like, ketones such as acetone, methyl ethyl ketone, methyl isobutyl ketone, cyclohexanone and the like, hydrocarbons such as hexane, heptane, octane, nonane and the like, halogenated hydrocarbons such as dichloromethane, chloroform, 1,2-dichloroethane and the like, organic acids such as formic acid, acetic acid, propionic acid and the like, polar solvents such as N,N-dimethylformamide, N,N-dimethyl acetamide, N-methylpyrrolidone and the like. These solvents can be used in a mixed solvent.
The used amount of the solvent is generally 1 to 10 times, and further preferably 3 to 7 times that of the indole compound.

The reaction temperature generally ranges from a low temperature less than room temperature to the boiling point of the solvent.
The reaction can be carried out by adding hydrobromic acid in a mixture of an indole compound, dimethyl sulfoxide and a solvent at a reaction temperature at a rate that dose not affect the reaction.
In the reaction, although sulfides formed by reduction of the sulfoxide are formed as by-products, the sulfides can be returned to sulfoxides with addition of an oxidizing agent that can oxidize sulfides to sulfoxides or by oxidation with oxygen, and the like. In this case, the regenerated sulfoxides can be re-used, and thus sulfoxides can be significantly reduced, and effects on the environment can be reduced.

The compound of formula (3) can be produced by reacting the indole compound of formula (2) directly with 3-chlorosulfonyl-1-(N,N-dimethylsulfamoyl)-1,2,4-triazole in the presence of a phase transfer catalyst and a base. 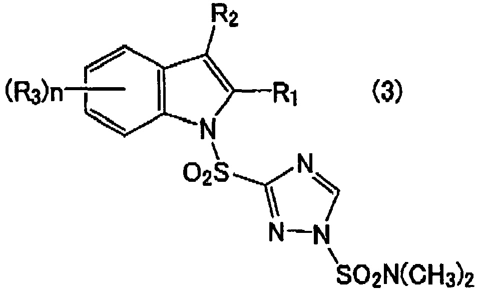 The compound of formula (3) is a fungicide disclosed in International Patent Publication WO99/21851 pamphlet.

The phase transfer catalyst includes quaternary ammonium salts such as tetramethyl ammonium chloride, tetramethyl ammonium bromide, tetramethyl ammonium iodide, tetramethyl ammonium hydroxide, tetraethyl ammonium chloride, tetraethyl ammonium bromide, tetraethyl ammonium iodide, tetrapropyl ammonium chloride, tetrapropyl ammonium bromide, tetrapropyl ammonium iodide, tetrabutyl ammonium chloride, tetrabutyl ammonium bromide, tetrabutyl ammonium iodide, tetrabutyl ammonium hydroxide, benzyl trimethyl ammonium chloride, benzyl triethyl ammonium chloride, benzyl tributyl ammonium chloride, trioctylmethyl ammonium chloride, phenyl trimethyl ammonium chloride or the like, quaternary phosphonium salts such as tetrabutyl phosphonium chloride, tetrabutyl phosphonium bromide, ethyltriphenyl phosphonium bromide, benzyl triphenyl phosphonium bromide, tetraphenyl phosphonium bromide or the like, pyridinium salts such as dodecyl pyridinium chloride or the like, crown ethers such as 15-crown-5-ether, dibenzo-18-crown-6-ether or the like. Tetrabutyl ammonium bromide, tetrabutyl ammonium hydrogensulfate or the like that is inexpensive and has a high reactivity, and is easy to remove after reaction is preferable.
The used amount of the phase transfer catalyst is generally 0.0001 to 1 mol, preferably 0.001 to 0.05 mol based on the indole compound.

The base includes alkali metal salts such as organic acid salt, carbonates, hydrogencarbonates, phosphates and sulfites or the like of alkali metal, alkaline earth metal salts such as organic acid salt, carbonates, hydrogencarbonates, phosphates and sulfites or the like of alkaline earth metal, hydroxides or oxides of alkali metal, and hydroxides or oxides of alkaline earth metal, and the like, preferably includes hydroxides of alkali metal such as potassium hydroxide or sodium hydroxide. Sodium hydroxide is preferable from the viewpoint of economic efficiency, particularly sodium hydroxide aqueous solution that is easy to handle is preferable.
The used amount of the base is preferably 0.1 to 10 mol, more preferably 1.0 to 3 mol based on the indole compound.

The used amount of 3-chlorosutfanyl-1-(N,N-dimethylsulfamoyl)-1,2,4-triazole is generally 0.5 to 3 mol, more preferably 1.0 to 1.5 mol based on the indole compound.

The solvent is not specifically limited so long as it is an inert solvent for the present reaction, and for example includes aromatic solvents such as benzene, toluene, xylene, chlorobenzene and the like, ethers such as diethyl ether, dibutyl ether, tetrahydrofuran, 1,4-dioxane, cyclopentyl methyl ether, dimethoxy ethane, diethylene glycol dimethyl ether and the like, esters such as methyl acetate, ethyl acetate, ethyl propionate and the like, halogenated hydrocarbons such as dichloromethane, chloroform, 1,2-dichloroethane and the like, ketones such as acetone, methyl ethyl ketone, methyl isobutyl ketone, cyclohexanone and the like, hydrocarbons such as hexane, heptane, octane, nonane and the like, polar solvents such as N,N-dimethylformamide, N,N-dimethyl acetamide, N-methylpyrrolidone and the like, pyridines such as pyridine, 2-methyl-5-ethyl pyridine, quinoline and the like, and water. These solvents can be used in a mixed solvent.
When sodium hydroxide aqueous solution is used as a base, the reaction is preferably carried out in the solvent separated with water among the above-mentioned solvents. The used amount of the solvent is generally 1 to 20 times, and further preferably 3 to 10 times that of the indole compound.

The reaction temperature generally ranges from a low temperature less than room temperature to the boiling point of the solvent.
The reaction can be carried out by reacting 3-chlorosulfonyl-1-(N,N-dimethylsulfamoyl)-1,2,4-triazole or a solution thereof with a mixture of an indole compound, a phase transfer catalyst, a base and a solvent, or by adding a base in a mixture of an indole compound, a phase transfer catalyst, 3-chlorosulfonyl-1-(N,N-dimethylsulfamoyl)-1,2,4-triazole and a solvent at a reaction temperature.

The compound of formula (3) can be isolated as crystal according to the following method. 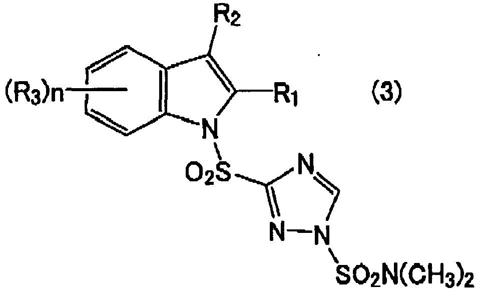 As solvents for crystallization, the above-mentioned solvents for the reaction can be used as such, and preferably the solvents for the reaction are subjected to concentration adjustment as such and used as solvents for crystallization. The solvents are preferably ethanol, 1,2-dichloraethane, toluene, xylene, chlorobenzene and the like.
The optimum solvent amount is selected by considering the yield and the state of the solution on crystallization, and the crystallization is preferably carried out in the solvent amount of 1 to 5 times.

In case of 1-(N,N-dimethylsulfamoyl)-3-(3-bromo-6-fluoro-2-methylindol-1-yl)sulfonyl-1,2,4 triazole, there are present as crystal polymorphism, an alpha-type crystal having a melting point of 132°C, a beta-type crystal having a melting point of 126°C, a gamma-type crystal having a transition point of 119°C to an alpha-type crystal, and the like, and a delta-type crystal being a pseudo crystal containing toluene, and the like.

Among them, the alpha-type crystal that has the highest melting point and is stable is desired as an aimed product. It can be produced by once obtaining a pseudo crystal containing a solvent such as toluene or chlorobenzene or the like, and then removing the solvent under reduced pressure or heating or the like, or by crystallizing in a solvent forming no pseudo crystal containing solvent. Preferably, the alpha-type crystal can be produced by reacting in toluene solvent, then adjusting the toluene amount to two to three times that of the aimed product, and filtering with cooling to obtain a delta-type crystal containing toluene, then removing toluene at a temperature of 40°C or more under reduced pressure.

If a little amount of the seed crystal of the delta-type crystal containing toluene is added on crystallization, only the delta-type crystal containing toluene can be reliably formed without formation of the beta-type crystal and gamma-type crystal, and at the same time, large delta-type crystals having a good filtering properties can be formed.

### Examples

Hereinafter, the present invention is further described according to examples to which the present invention is not limited.

### Example 1

### Production of 6-fluoro-2-methylindole

In a reaction flask that the atmosphere was substituted by nitrogen, 200 g (1.01 mol) of 4-fluoro-2-nitrophenyl acetone, 1060 g of toluene, 41.6 g (0.507 mol) of sodium acetate, 207.1 g (2.03 mol) of acetic anhydride and 170 g (3.04 mol) of iron powder were placed, and the temperature was raised to 100°C, and 487 g (8.12 mol) of acetic acid was added dropwise over 3 hours, then the resulting mixture was reacted for 1.5 hour. After confirming disappearance of 4-fluoro-2-nitrophenyl acetone with liquid chromatography, 1600 g of water was added and cooled to room temperature. After adding 207 g of 97% sulfuric acid, insoluble iron oxide and unreacted iron were removed by filtration. The toluene phase was separated, then washed with 400 g of water, a mixed solution of 400 g of water and 40 g of 30% sodium hydroxide aqueous solution, and washed with 400 g of water two times to obtain a solution of 6-fluoro-2-methylindole in toluene. A quantitative analysis with liquid chromatography confirmed that 144.4 g (yield 95.4%) of 6-fluoro-2-methylindole was formed.

### Example 2

### Production of 6-fluoro-2-methylindole

In a reaction flask that the atmosphere was substituted by nitrogen, 20.0 g (0.101 mol) of 4-fluoro-2-nitrophenyl acetone, 106 g of toluene, 4.16 g (0.0507 mol) of sodium acetate, 20.7 g (0.203 mol) of acetic anhydride and 17.0 g (0.304 mol) of iron powder were placed, and the temperature was raised to 100°C, and 30.5 g (0.507 mol) of acetic acid was added dropwise over 1 hour, then the resulting mixture was reacted for 4 hours. After confirming disappearance of 4-fluoro-2-nitrophenyl acetone with liquid chromatography, 160 g of water was added and cooled to room temperature. After insoluble iron oxide and unreacted iron were removed by filtration, the toluene phase was separated, then washed with 40 g of water, a mixed solution of 40 g of water and 1.6 g of 30% sodium hydroxide aqueous solution, and washed with 40 g of water two times to obtain a solution of 6-fluoro-2-methylindole in toluene. A quantitative analysis with liquid chromatography confirmed that 14.0 g (yield 92.4%) of 6-fluoro-2-methylindole was formed. After distilling off to 10.5 g of toluene at 70°C under reduced pressure, the solution in which 24.5 g of heptane was added was cooled to 0°C to be crystallized. The resulting crystal was washed with 14.0 g of heptane to obtain 12.1 g of 6-fluoro-2-methylindole as crystal.

### Example 3

### Production of 3-bromo-6-fluoro-2-methylindole

In a solution of 40.0 g (0.268 mol) of 6-fluoro-2-methylindole in 200 g of toluene, 33.5 g (0.429 mol) of dimethylsulfoxide was added under nitrogen atmosphere, and the temperature was adjusted to 20°C. In this solution, 73.9 g (0.429 mol) of 47% hydrogen bromide was added dropwise at 18 to 22°C over 1 hour and the resulting mixture was reacted at 18 to 22°C further for 7 hours. After the confirmation of the formation of the aimed product with HPLC, the reaction solution was cooled to 5 to 10°C, 120 g of water was added dropwise, and subjected to phase-separation, then washed with 120 g of water at 5 to 10°C two times to obtain a solution of 3-bromo-6-fluoro-2-methylindole in toluene.

### Example 4

### Production of 1-(N,N-dimethylsulfamoyl)-3-(3-bromo-6-fluoro-2-methylindol-1-yl) sulfonyl-1,2,4-triazole

In the solution of 3-bromo-6-fluoro-2-methylindole in toluene prepared in the preceding step, 53.6 g (0.402 mol) of 30% sodium hydroxide and 0.865 g (0.00268 mol) of tetrabutyl ammonium bromide were added at 0 to 5°C under nitrogen atmosphere, then a solution of 87.2 g (0.317 mol) of 3-chlorosulfonyl-1-(N,N-dimethylsulfamoyl)-3-chlorosulfonyl-1,2,4-triazole in 419 g of toluene separately prepared by dissolving at 50°C under nitrogen atmosphere was added dropwise at -5 to 0°C over 4 hours, and stirred at 0°C further for 3 hours. After the completion of the reaction, 183 g of water was added, the temperature was raised to 40°C, the resulting solution was subjected to phase-separation, and then washed with 183 g of water two times to obtain a solution of 118.7 g (yield 95.0%) of 1-(N,N-dimethylsulfamoyl)-3-(3-bromo-6-fluoro-2-methylindol-1-yl) sulfonyl-1,2,4-triazole being an aimed product in toluene. After reducing the amount of toluene by distilling off the toluene to 2.5 times that of the aimed product, crystal was separated out by cooling to -5 to 0°C. The crystal was filtered, washed with 113 g of cooled toluene to obtain delta-type crystal containing toluene, and then dried at 60°C under reduced pressure to obtain 106.4 g (yield 85.1 %) of alpha-type crystal of 1-(N,N-dimethylsulfamoyl)-3-(3-bromo-6-fluoro-2-methylindol-1-yl) sulfonyl-1,2,4-triazole.

### Example 5

### Production of 3-bromo-6-fluoro-2-methylindole

In a solution of 232.3 g (1.558 mol) of 6-fluoro-2-methylindole in 1162 g of toluene, 133.9 g (1.713 mol) of dimethylsulfoxide was added under nitrogen atmosphere, and the temperature was adjusted to 20°C. In this solution, 455.9 g (2.648 mol) of 47% hydrogen bromide was added dropwise at 18 to 22°C over 2 hours and stirred at 18 to 22°C further for 7 hours. After the confirmation of the formation of the aimed product with HPLC, the reaction solution was cooled to 5 to 10°C and 604 g of water and 160 g of 30% sodium hydroxide were added dropwise, and then subjected to phase-separation, then washed with 604 g of water two times to obtain a solution of 3-bromo-6-fluoro-2-methylindole in toluene.

### Example 6

### Production of 1-(N,N-dimethylsulfamoyl)-3-(3-bromo-6-fluoro-2-methylindol-1-yl) sulfonyl-1,2,4-triazole

In the solution of 3-bromo-6-fluoro-2-methylindole in toluene prepared in the preceding step, 311.5 g (2.336 mol) of 30% sodium hydroxide and 5.02 g (0.0156 mol) of tetrabutyl ammonium bromide were added at 0 to 5°C under nitrogen atmosphere, then a solution of 511.2 g (1.860 mol) of 3-chlorosulfonyl-1-(N,N-dimethylsulfamoyl)-3-chlorosulfonyl-1,2,4-triazole in 2986 g of toluene separately prepared by dissolving at 50°C under nitrogen atmosphere was added dropwise at -5 to 0°C over 4 hours, and stirred at 0°C further for 3 hours. After the completion of the reaction, 1066 g of water was added, the temperature was raised to 40°C, the resulting mixture was subjected to phase-separation. Thereafter, in order to remove dimethyl sulfide contaminating from the preceding step, 1066 g of water, 194.2 g (1.713 mol) of 30% hydrogen peroxide and 92.6 g of 35% sodium bisulfite were added in that order and treated. After subjecting to phase-separation, the toluene phase was washed with 1066 g of water two times to obtain a solution of 657.3 g (yield 90.5%) of 1-(N,N-dimethylsulfamoyl)-3-(3-bromo-6-fluoro-2-methylindol-1-yl) sulfonyl-1,2,4-triazole being an aimed product in toluene. After reducing the amount of toluene by distilling off the toluene to 2.5 times that of the aimed product at 60°C, the resulting solution was subjected to crystallization by cooling gradually and adding a little amount of crystal containing toluene from 55°C at 1°C intervals, and then it was cooled to -5 to 0°C. The crystal was filtered, washed with 657 g of cooled toluene to obtain delta-type crystal containing toluene, and then dried at 60°C under reduced pressure to obtain 598.4 g (yield 79.5%) of alpha-type crystal of 1-(N,N-dimethylsulfamoyl)-3-(3-bromo-6-fluoro-2-methylindol-1-yl) sulfonyl-1,2,4-triazole.

### Example 7

### Production of 6-fluoro-2-methylindole

In a reaction flask that the atmosphere was substituted by nitrogen, 422 g of toluene, 150 g (2.68 mol) of iron powder and 43.9 g (1.34 mol) of sodium acetate were placed, and the temperature was raised to 90°C, and 273 g (2.68 mol) of acetic anhydride and 642 g (10.7 mol) of acetic acid were added dropwise. In the solution, a solution 211 g (1.07 mol) of 4-fluoro-2-nitrophenyl acetone in 422 g of toluene was added dropwise at 90°C over 3 hours. After the completion of the addition, the temperature was raised to 100°C and subjected to reaction for 3 hours. After confirming disappearance of 4-fluoro-2-nitrophenyl acetone with liquid chromatography, 1688 g of water was added and cooled to room temperature. After adding 223 g of 97% sulfuric acid dropwise, insoluble iron oxide and unreacted iron were removed by filtration. After separating the toluene phase, it was washed with 400 g of water, with a mixed solution of 400 g of water and 40 g of 30% sodium hydroxide aqueous solution and with 400 g of water two times to obtain a solution of 6-fluoro-2-methylindole in toluene. A quantitative analysis with liquid chromatography confirmed that 149 g (yield 93.2%) of 6-fluoro-2-methylindole was formed.

### Example 8

### Production of 1-(N,N-dimethylsulfamoyl)-3-(3-bromo-6-fluoro-2-methylindol-1-yl) sulfonyl-1,2,4-triazole

In the solution of 3-bromo-6-fluoro-2-methylindole in toluene prepared from 7.00 g (0.0469 mol) of 6-fluoro-2-methylindole in the preceding step, 14.2 g (0.0516 mol) of 3-chlorosuffonyl-1-(N,N-dimethylsulfamoyl)-3-chlorosulfonyl-1,2,4-triazole in 85.1 g of toluene and 0.151 g (0.000469 mol) of tetrabutyl ammonium bromide were added, and 5.99 g (0.0704 mol) of 48% sodium hydroxide was added dropwise at -5 to 0°C over 4 hours, and stirred at -5 to 0°C further for 3 hours. After the completion of the reaction, 32 g of water was added, the temperature was raised to 40°C, the resulting mixture was subjected to phase-separation, and then washed with 32 g of water two times to obtain a solution of 20.0 g (yield 91.2%) of 1-(N,N-dimethylsulfamoyl)-3-(3-bromo-6-fluoro-2-methylindol-1-yl) sulfonyl-1,2,4-triazole being an aimed product in toluene.

### Reference Example 1

### Production of bis[1-(N,N-dimethylsulfamoyl)-1,2,4-triazole-3-yl]disulfide

In a mixture of 327.1 g (1.634 mol) of bis[1,2,4-triazole-3-yl]disulfide and 1636 g of 1,2-dichloroethane, 19.0 g (0.1634 mol) of N,N,N',N'-tetramethyl ethylene diamine and 346.3 g (3.268 mol) of sodium carbonate were added, the temperature was raised to 30°C, and 492.6 g (3.431 mol) of N,N-dimethylsulfamoyl chloride was added dropwise at a temperature between 28°C to 32°C over 2 hours, reacted at a temperature between 28°C and 32°C for 6 hours. After the completion of the reaction, 2944 g of 1,2-dichloroethane was added, and the resulting solution was added in a mixture of 340.7 g of 35% hydrochloric acid and 3925 g of water at a temperature ranging from 20°C to 25°C. The aqueous phase was separated out to obtain 5548 g of a 1,2-dichloroethane solution containing 629.1 g of bis[1-(N,N-dimethylsulfamoyl)-1,2,4-triazole-3-yl]disulfide (yield: 92.9% by quantitative analysis with liquid chromatography).

### Reference Example 2

### Production of 3-chlorosulfonyl-1-(N, N-dimethylsulfamoyl)-1,2,4-triazole

In 4800 g of a 1,2-dichloroethane solution containing 600 g (1.45 mol) of bis[1-(N,N-dimethylsulfamoyl)-1,2,4-triazole-3-yl]disulfide, 1800 g of water was added, cooled to 15°C, 300 g of methanol was added and then 564.5 g (7.96 mol) of chlorine gas was blown at a temperature ranging from 15°C to 20°C over 3 hours. Thereafter, the resulting mixture was reacted at 15 to 20°C for 0.5 hour. After the completion of the reaction, the solution was subjected to phase-separation, washed with 1620 g of water three times to obtain a 1,2-dichloroethane solution containing 724.3 g (yield: 91.1% by analysis with liquid chromatography) of 3-chlorosulfonyl-1-(N,N-dimethylsulfamoyl)-1,2,4-triazole.

### Reference Example 3

### Production of 1-(4 fluoro-2-nitrophenyl) acetone

In a mixed solution of 15.0 g (0.0943 mol) of 2,5-difluoronitrobenzene and 30 g of dimethylsulfoxide, 39.1g (0.283 mol) of potassium carbonate was added, the temperature was raised to 50°C; 11.3 g (0.113 mol) of acetyl acetone was added dropwise at room temperature ranging from 48°C to 52°C over 1 hour. After the reaction was carried out at 48 to 52°C for 9.5 hours, 90.0 g of toluene, 30.0 g of water and 30.0 g of methanol were added, the resulting mixture was reacted at a temperature ranging from 48°C to 52°C for 9 hours. After the completion of the reaction, the solution was cooled to room temperature, 120 g of water was added and subjected to phase-separation, washed with 30 g of water two times to obtain a toluene solution containing 16 g (yield: 86.2% from 2,5-difluoronitrobenzene by quantitative analysis with liquid chromatography) of 1-(4-fluoro-2-nitrophenyl) acetone.

### Industrial Applicability

According to the process of the present invention, 6-fluoro-2-methylindole that is a useful intermediate for production of indole compounds and that has been difficult to produce at a low cost in the prior art can be easily obtained from 2-nitrophenyl acetones, and further 3-halogenated forms and 1-sulfonyl forms of the indole compounds can be produced by an inexpensive and simple method. Therefore, the process of the present Invention is very excellent as an industrial production process.

## Claims

1. A process for producing an indole compound of formula (2-2) 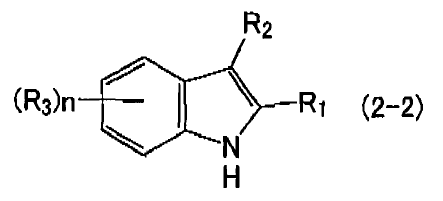 wherein R₁ is a hydrogen atom, a halogen atom, an optionally substituted alkyl group, or a phenyl group. R₂ is a bromine atom, R₃ is an optionally substituted alkyl group, a phenyl group, an alkoxy group, an alkoxycarbonyl group, or a halogen atom, and n is an integer of 0 to 4,
comprising reacting an indole compound of formula (2-1) 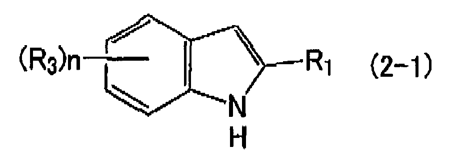 wherein R₁, R₃ and n have the same meaning as above,
with a hydrogen halide acid and a sulfoxide compound, wherein
the hydrogen halide acid is hydrobromic acid and
the sulfoxide compound is dimethylsulfoxide.

2. The process for producing an indole compound of formula (2-2) according to claim 1, further comprising reducing a 2-nitrobenzylcarbonyl compound of formula (1) 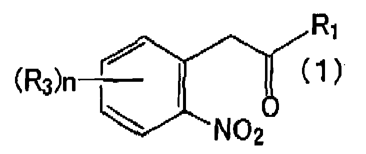 wherein R₁, R₃ and n have the same meaning as in claim 1,
with a metal and an acid in the presence of an acylating agent and a base to produce the indole compound of formula (2-1).

3. A process for producing a sulfamoyl triazole compound of formula (3) 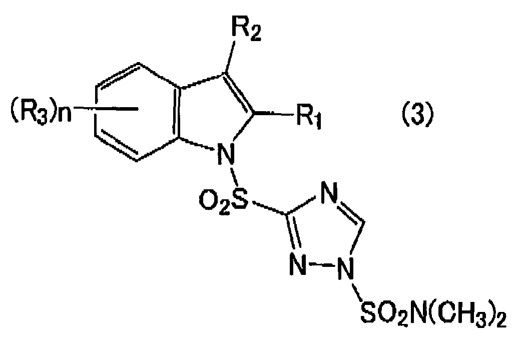 wherein R₁ is a hydrogen atom, a halogen atom, an optionally substituted alkyl group, or a phenyl group. R₂ is a bromine atom, R₃ is an optionally substituted alkyl group, a phenyl group, an alkoxy group, an alkoxycarbonyl group, or a halogen atom, and n is an integer of 0 to 4,
comprising producing the indole compound of formula (2-2) by the process according to claim 1 or claim 2 and reacting the indole compound of formula (2-2) directly with 3-chlorosulfonyl-1-(N,N-dimethylsulfamoyl)-1,2,4-triazole in the presence of a phase transfer catalyst and a base.

4. The process for producing an indole compound (2-2) according to claim 2, wherein the acylating agent is an organic acid anhydride

5. The process for producing an indole compound (2-2) according to claim 2, wherein the acylating agent is acetic anhydride.

6. The process for producing an indole compound (2-2) according to claim 2, wherein the base is an alkali metal salt or a hydroxide of alkali metal.

7. The process for producing an indole compound (2-2) according to claim 2, wherein the base is an organic acid salt, carbonate, hydrogencarbonate or hydroxide of alkali metal.

8. The process for producing an indole compound (2-2) according to claim 2, wherein the metal is iron.

9. The process for producing an indole compound according to claim 1 or claim 2, wherein the indole compound of formula (2-2) is 3-bromo-6-fluoro-2-methylindole.

10. A process for producing 3-bromo-6-fluoro-2-methylindole, **characterized by** reacting 2-methyl-6-fluoroindole with hydrobromic acid and a sulfoxide compound.

## Patentansprüche

1. Verfahren zur Herstellung einer Indolverbindung der Formel (2-2): 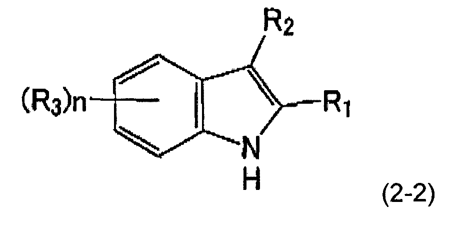 worin R₁ ein Wasserstoffatom, ein Halogenatom, eine gegebenenfalls substituierte Alkylgruppe oder eine Phenylgruppe ist, R₂ ein Bromatom ist, R₃ eine gegebenenfalls substituierte Alkylgruppe, eine Phenylgruppe, eine Alkoxygruppe, eine Alkoxycarbonylgruppe oder ein Halogenatom ist und n eine ganze Zahl von 0 bis 4 ist,
umfassend das Umsetzen einer Indolverbindung der Formel (2-1): 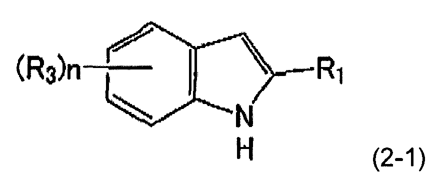 worin R₁, R₃ und n die gleichen Bedeutungen wie oben haben,
mit einer Halogenwasserstoffsäure und einer Sulfoxidverbindung,
wobei die Halogenwasserstoffsäure Bromwasserstoffsäure ist und die Sulfoxidverbindung Dimethylsulfoxid ist.

2. Verfahren zur Herstellung einer Indolverbindung der Formel (2-2) gemäss Anspruch 1, das ferner das Reduzieren einer 2-Nitrobenzylcarbonylverbindung der Formel (1): 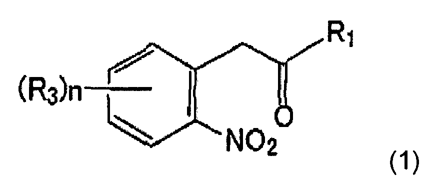 worin R₁, R₃ und n die gleichen Bedeutungen haben wie in Anspruch 1 haben,
mit einem Metall und einer Säure in Gegenwart eines Acylierungsmittels und einer Base umfasst, um eine Indolverbindung der Formel (2-1) herzustellen.

3. Verfahren zur Herstellung einer Sulfamoyltriazolverbindung der Formel (3): 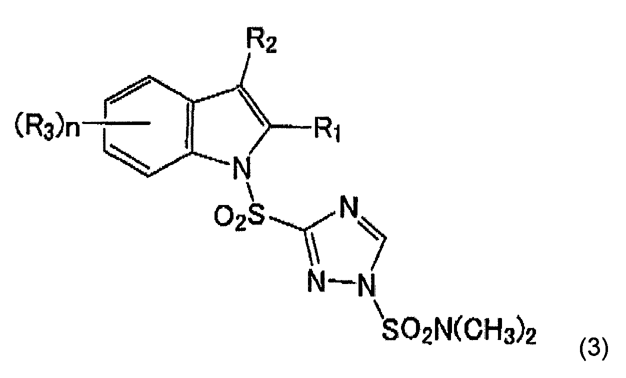 worin R₁ ein Wasserstoffatom, ein Halogenatom, eine gegebenenfalls substituierte Alkylgruppe oder eine Phenylgruppe ist, R₂ ein Bromatom ist, R₃ eine gegebenenfalls substituierte Alkylgruppe, eine Phenylgruppe, eine Alkoxygruppe, eine Alkoxycarbonylgruppe oder ein Halogenatom ist und n eine ganze Zahl von 0 bis 4 ist,
das die Herstellung einer Indolverbindung der Formel (2-2) nach einem Verfahren gemäss Anspruch 1 oder Anspruch 2 und die direkte Umsetzung der Indolverbindung der Formel (2-2) mit 3-Chlorsulfonyl-1-(N,N-dimethylsulfamoyl)-1,2,4-triazol in Gegenwart eines Phasentransferkatalysators und einer Base umfasst.

4. Verfahren zur Herstellung einer Indolverbindung (2-2) gemäss Anspruch 2, wobei das Acylierungsmittel ein organisches Säureanhydrid ist.

5. Verfahren zur Herstellung einer Indolverbindung (2-2) gemäss Anspruch 2, wobei das Acylierungsmittel Essigsäureanhydrid ist.

6. Verfahren zur Herstellung einer Indolverbindung (2-2) gemäss Anspruch 2, wobei die Base ein Alkalimetallsalz oder ein Hydroxid eines Alkalimetalls ist.

7. Verfahren zur Herstellung einer Indolverbindung (2-2) gemäss Anspruch 2, wobei die Base ein organisches Säuresalz, Carbonat, Hydrogencarbonat oder ein Hydroxid eines Alkalimetalls ist.

8. Verfahren zur Herstellung einer Indolverbindung (2-2) gemäss Anspruch 2, wobei das Metall Eisen ist.

9. Verfahren zur Herstellung einer Indolverbindung gemäss Anspruch 1 oder Anspruch 2, wobei die Indolverbindung der Formel (2-2) 3-Brom-6-fluor-2-methylindol ist.

10. Verfahren zur Herstellung von 3-Brom-6-fluor-2-methylindol, **gekennzeichnet durch** die Umsetzung von 2-Methyl-6-fluorindol mit Bromwasserstoffsäure und einer Sulfoxidverbindung.

## Revendications

1. Procédé de production d'un composé indole de
formule (2-2) 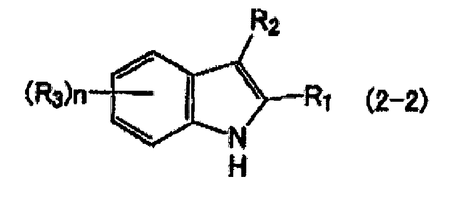 dans laquelle R₁ est un atome d'hydrogène, un atome d'halogène, un groupe alkyle éventuellement substitué, ou un groupe phényle. R₂ est un atome de brome, R₃ est un groupe alkyle éventuellement substitué, un groupe phényle, un groupe alcoxy, un groupe alcoxycarbonyle, ou un atome d'halogène, et n est un nombre entier de 0 à 4,
comprenant la réaction d'un composé indole de formule (2-1) 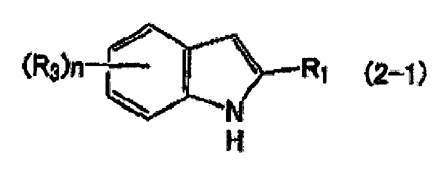 dans laquelle R₁, R₃ et n ont la même signification que ci-dessus,
avec un acide halohydrique et un composé sulfoxyde, dans lequel
l'acide halohydrique est l'acide bromhydrique et
le composé sulfoxyde est le diméthylsulfoxyde.

2. Procédé de production d'un composé indole de formule (2-2) selon la revendication 1, comprenant en outre la réduction d'un composé 2-nitrobenzylcarbonyle de formule (1) 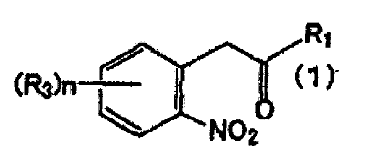 dans laquelle R₁, R₃ et n ont la même signification que dans la revendication 1,
avec un métal et un acide en présence d'un agent d'acylation et d'une base pour produire le composé indole de formule (2-1).

3. Procédé de production d'un composé sulfamoyl triazole de formule (3) 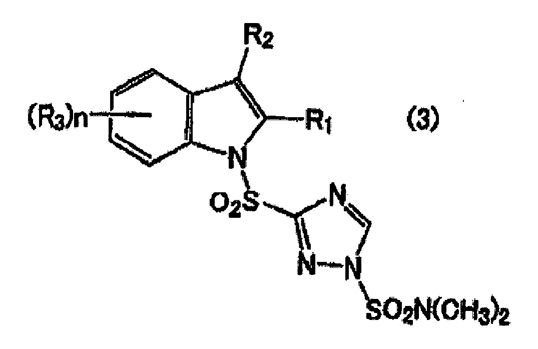 dans laquelle R₁ est un atome d'hydrogène, un atome d'halogène, un groupe alkyle éventuellement substitué, ou un groupe phényle. R₂ est un atome de brome, R₃ est un groupe alkyle éventuellement substitué, un groupe phényle, un groupe alcoxy, un groupe alcoxycarbonyle, ou un atome d'halogène, et n est un nombre entier de 0 à 4,
comprenant la production du composé indole de formule (2-2) par le procédé selon la revendication 1 ou la revendication 2 et la réaction du composé indole de formule (2-2) directement avec le 3-chlorosulfonyl-1-(N,N-diméthylsulfamoyl)-1,2,4-triazole en présence d'un catalyseur de transfert de phase et d'une base.

4. Procédé de production d'un composé indole (2-2) selon la revendication 2, dans lequel l'agent d'acylation est un anhydride d'acide organique

5. Procédé de production d'un composé indole (2-2) selon la revendication 2, dans lequel l'agent d'acylation est l'anhydride acétique.

6. Procédé de production d'un composé indole (2-2) selon la revendication 2, dans lequel la base est un sel de métal alcalin ou un hydroxyde de métal alcalin.

7. Procédé de production d'un composé indole (2-2) selon la revendication 2, dans lequel la base est un sel d'acide organique, un carbonate, un hydrogénocarbonate ou un hydroxyde de métal alcalin.

8. Procédé de production d'un composé indole (2-2) selon la revendication 2, dans lequel le métal est le fer.

9. Procédé de production d'un composé indole selon la revendication 1 ou la revendication 2, dans lequel le composé indole de formule (2-2) est le 3-bromo-6-fluoro-2-méthylindole.

10. Procédé de production de 3-bromo-6-fluoro-2-méthylindole, **caractérisé par** la réaction de 2-méthyl-6-fluoroindole avec de l'acide bromhydrique et un composé sulfoxyde.
